# EUROPÄISCHE PATENTSCHRIFT

(11) **EP 1 948 789 B1**
(45) Veröffentlichungstag und Bekanntmachung des Hinweises auf die Patenterteilung: **09.06.2010**
(21) Anmeldenummer: 06818078.5
(22) Anmeldetag: 15.11.2006
(51) Int. Cl.: C12N 5/071, C12N 5/10

(54) **VERFAHREN ZUR HERSTELLUNG VON PERMANENTEN HUMANEN ZELLLINIEN**
METHOD FOR THE PRODUCTION OF PERMANENT HUMAN CELL LINEAGES
PROCEDE DE PRODUCTION DE LIGNEES CELLULAIRES HUMAINES PERMANENTES

(30) Priorität: 16.11.2005 DE 102005054628
(43) Veröffentlichungstag der Anmeldung: 30.07.2008
(73) Patentinhaber: CEVEC Pharmaceuticals GmbH, 51105 Köln (DE)
(72) Erfinder: SCHIEDNER, Gudrun, 51105 Köln (DE); VOLPERS, Christoph, 51105 Köln (DE)
(74) Vertreter: Dehmel, Albrecht
(86) Internationale Anmeldenummer: PCT/DE2006/002004
(87) Internationale Veröffentlichungsnummer: WO 2007/056994

(56) Entgegenhaltungen:
- WO-A-02/40665
- US-B1- 6 558 948
- JENKINS E C ET AL: "SV-40-TRANSFORMED FRAGILE X AMNIOCYTES" AMERICAN JOURNAL OF MEDICAL GENETICS, Bd. 38, Nr. 2-3, 1991, Seiten 464-466, XP002423695 ISSN: 0148-7299
- WALEN K H: "CHROMOSOME INSTABILITY IN CELL LINEAGES OF AMNIOCYTE CLONES MORPHOLOGICALLY TRANSFORMED BY SV-40" CANCER GENETICS AND CYTOGENETICS, Bd. 25, Nr. 1, 1987, Seiten 149-160, XP002423696 ISSN: 0165-4608
- WALEN K H ET AL: "INDUCTION OF TUMORGENESIS AND CHROMOSOMAL ABNORMAILITIES IN HUMAN AMNIOCYTES INFECTED WITH SIMIAN VIRUS 40 AND KIRSTEN SARCOMA VIRUS" IN VITRO CELLULAR AND DEVELOPMENTAL BIOLOGY, THE ASSOCIATION, GAITHERSBURG, MD, US, Bd. 22, Nr. 2, Februar 1986 (1986-02), Seiten 57-65, XP000939354 ISSN: 0883-8364
- JENKINS L S ET AL: "TRANSFORMATION AND ESTABLISHMENT OF FRAGILE X CELL LINES FROM AMNIOCYTES" AMERICAN JOURNAL OF MEDICAL GENETICS, Bd. 38, Nr. 2-3, 1991, Seiten 416-417, XP002423697 ISSN: 0148-7299
- JONES D H ET AL: "PER.C6 CELL LINE FOR HUMAN ANTIBODY PRODUCTION CRUCELL'S TECHNOLOGY MAINTAINS HUMAN GLYCOSYLATION PATTERNS" GENETIC ENGINEERING NEWS, MARY ANN LIEBERT, NEW YORK, US, Bd. 22, Nr. 10, 15. Mai 2002 (2002-05-15), Seite 50,54, XP009018783 ISSN: 1270-6377
- HUANG Z ET AL: "Enhancing protein expression in single HEK 293 cells" JOURNAL OF NEUROSCIENCE METHODS, ELSEVIER SCIENCE PUBLISHER B.V., AMSTERDAM, NL, Bd. 142, Nr. 1, 15. März 2005 (2005-03-15), Seiten 159-166, XP004714421 ISSN: 0165-0270
- MAY TOBIAS ET AL: "Application of a reversible immortalization system for the generation of proliferation-controlled cell lines" CYTOTECHNOLOGY, Bd. 46, Nr. 2-3, Oktober 2004 (2004-10), Seiten 69-78, XP002423750 ISSN: 0920-9069
- SCHIEDNER G ET AL: "EFFICIENT TRANSFORMATION OF PRIMARY HUMAN AMNIOCYTES BY E1 FUNCTIONS OF AD5: GENERATION OF NEW CELL LINES FOR ADENOVIRAL VECTOR PRODUCTION" HUMAN GENE THERAPY, MARY ANN LIEBERT, NEW YORK ,NY, US, Bd. 11, Nr. 15, 10. Oktober 2000 (2000-10-10), Seiten 2105-2116, XP000974022 ISSN: 1043-0342
- JANABI N ET AL: "ESTABLISHMENT OF HUMAN MICROGLIAL CELL LINES AFTER TRANSFECTION OF PRIMARY CULTURES OF EMBRYONIC MICROGLIAL CELLS WITH THE SV40 LARGE T ANTIGEN" NEUROSCIENCE LETTERS, LIMERICK, IE, Bd. 195, 1995, Seiten 105-108, XP002938786 ISSN: 0304-3940

### SEQUENCE LISTING

<110> CEVEC Pharmaceuticals GmbH
<120> Verfahren zur Herstellung von permanenten humanen Zelllinien
<130> CMV-011 PCT
<140> unknown
   <141> 2006-11-15
<150> DE 10 2005 054 628.5
   <151> 2005-11-16
<160> 4
<170> PatentIn version 3.3
<210> 1
   <211> 30
   <212> DNA
   <213> artificial sequence
<220>
   <223> Oligonucleotide primer
<400> 1
   ctggctcgag ctctagcgat gaagatacag 30
<210> 2
   <211> 30
   <212> DNA
   <213> artificial sequence
<220>
   <223> Oligonucleotide primer
<400> 2
   gctgctcgag cacttgcttg atccaaatcc 30
<210> 3
   <211> 56 <212> DNA
   <213> artificial sequence
<220>
   <223> Oligonucleotide primer
<400> 3
   gctgggcgtg gtgcctaaaa atgtctttca gtagcaagct gattgccagt ttaaac 56
<210> 4
   <211> 61
   <212> DNA
   <213> artificial sequence
<220>
   <223> Oligonucleotide primer
<400> 4
   tcgagtttaa actggcaatc agcttgctac tgaaagacat ttttaggcac cacgcccagc 60
   t 61

## Beschreibung

Die vorliegenden Erfindung betrifft ein Verfahren zur Herstellung einer permanenten humanen Zelllinie, wobei isolierte primäre humane Zellen gleichzeitig mit einer Sequenz, die die Expression von mindestens einem Zell-transformierenden Faktor erlaubt und einer Sequenz, die die Expression mindestens eines rekombinanten Polypeptids erlaubt, transfiziert werden.

Die Produktion von rekombinanten Polypeptiden für therapeutische, diagnostische oder technische Zwecke in Zellkultur (in vitro) erfolgt in der Regel in stabilen, dauerhaft oder permanent etablierten Zelllinien, in denen die Nukleinsäure, die das Polypeptid codiert, in die chromosomale DNA der Zelle integriert ist (sog. Produktionszelllinie). Diese Produktionszelllinien müssen insbesondere zwei charakteristische Eigenschaften aufweisen: erstens müssen sie dauerhaft (permanent) in Zellkultur wachsen und vermehrbar sein, und zweitens müssen sie das Gen exprimieren, das das gewünschte Polypeptid codiert, welches dann aus der Zelle oder aus dem Kulturüberstand isoliert werden kann. Neben Bakterien, Hefen und Pflanzenzelle werden insbesondere tierische Zellen zur Produktion rekombinanter Polypeptide verwendet. Etwa 60-70% aller therapeutischen Proteine werden heute in Säugerzellen produziert (Wurm, Nat. Biotechnology 22, 1393-1398, 2004). Die Herstellung der Produktionszellen geht in der Regel von bereits immortalisierten oder transformierten Zelllinien aus, die dauerhaft in Zellkultur wachsen und sich vermehren, wie beispielsweise tierische CHO (Chinese Hamster Ovary)-, BHK (Baby Hamster Kidney)-, NS0 (Maus Myelom)-Zellen oder menschliche HEK293 (Human Embryonic Kidney)-, oder PER.C6-Zellen. Diese Zelllinien, die inzwischen auch kommerziell erhältlich sind, wurden quasi als erster Schritt des Verfahrens zur Etablierung von Produktionszellen durch genetische Manipulation aus primären Zellen in Kultur generiert oder aus einem natürlichen Tumor isoliert.

Bei der Gewinnung der eigentlichen Produktionszellen wird durch Transfektion in diese bereits etablierten Zelllinien zum einen die Nukleinsäure, die das rekombinante Polypeptid codiert (das sog. Transgen), mit den notwendigen transkriptionellen Regulationselementen transferiert, zum anderen eine zweite Expressionskassette mit einem Gen, das einen Selektionsmarker codiert und dessen Genprodukt der Zelle einen bestimmten Selektionsvorteil verschafft. Wenige Tage nach dem Gentransfer, während denen die Zellen in einem Kulturmedium ohne Selektionsreagenz kultiviert werden, wird dem Medium ein geeignetes Selektionsreagenz zugesetzt. In Gegenwart der Selektionsreagenz überleben und wachsen nur die Zellen, die die zur Transfektion benutzten Nukleinsäuren aufgenommen haben und den Selektionsmarker exprimieren. Häufig benutzte Selektionsmarker sind das Neomycin-Resistenzgen, das Hygromycin-Resistenzgen und die Dihydrofolat-Reduktase (DHFR)(Wurm, Nat. Biotechnology 22:1393-1398, 2004; Wurm und Jordan, 309-333 in: Makrides (Hrsg.), Gene Transfer and Expression in Mammalian Cells, Elsevier, Amsterdam, 2003). Die Selektion erfolgt entsprechend in Kulturmedium mit den Selektionsreagenzien wie den Antibiotika Neomycin oder Hygromycin bzw. dem synthetischen Glukokortikoid Methotrexat. Zellen mit dem Selektionsmarker und dem Transgen, die den Selektionsprozess überleben und proliferieren (sog. Transformanten), werden in der Regel anschließend vereinzelt (kloniert), um sicherzustellen, dass alle Zellen in der Kultur genetisch identisch sind und um die gewünschten Produktionszelllinien mit der besten Produktionsrate von weniger gut produzierenden Zelllinien zu trennen.

Die Selektionsreagenzien müssen, je nach Herstellungsprozess, unter Umständen nicht nur während der Selektionsphase, sondern auch später bei der Anzucht der klonierten Zellen dem Medium zugesetzt werden, um die genetische Stabilität der Produktionszellen sicherzustellen. Das Gen für das gewünschte Polypeptid (Transgen) und das Gen für den Selektionsmarker integrieren in der Regel an derselben Stelle im zellulären Genom. Bei Fehlen des Selektionsdrucks während der späteren Expansion der Zellen oder während der Produktionsphase besteht die Gefahr, dass die Zellen den Selektionsmarker - und damit auch das Transgen für das gewünschte Polypeptid - verlieren. Dies wird durch den kontinuierlichen Zusatz der Selektionsreagenz verhindert.

Primäre tierische und menschliche Zellen, die nach Entnahme aus dem Organismus oder Gewebe in Zellkultur gebracht werden, wachsen in der Regel nur über kurze Zeit und wenige Passagen. Humane Zellen sind für die Herstellung humaner Biotherapeutika besonders gut geeignet, da sie komplexe Polypeptide - im Gegensatz zu anderen Säugerzellen oder tierischen Zellen - mit authentischem posttranslationalem Modifikationsmuster exprimieren. Das Glykosylierungsmuster komplexer rekombinanter Proteine, also die Struktur und Anordnung der Zuckerreste im Molekül, wird bei einer Herstellung in humanen Zellen wesentlich besser das Muster des authentischen humanen Polypeptids reproduzieren als bei einer Herstellung in nichthumanen Produktionssystemen. Dieses Glykosylierungsmuster ist häufig für wichtige Eigenschaften des Polypeptids wie biologische Aktivität, Stabilität, Löslichkeit und Immunogenität von entscheidender Bedeutung.

Dauerhaft etablierte humane Zelllinien (permanente humane Zelllinien), die für die Expression rekombinanter Polypeptide verwendet werden könnten, wurden aus Tumorgewebe von Patienten isoliert (z.B. HeLa-Zellen). Solche Zellen sind aber aus sicherheitsrelevanten Erwägungen und auf Grund ihrer genetischen Instabilität für eine industrielle Produktion von therapeutischen Polypeptiden zur Anwendung am Menschen nicht geeignet. Spontan immortalisierte humane Zelllinien sind mit wenigen Ausnahmen (z.B. die sog. HaCaT-Zellen) praktisch nicht verfügbar. Geeignet für biotechnologische Produktionszwecke sind dagegen permanente humane Zelllinien, die durch Transformation primären Gewebes mittels bestimmter viraler Genprodukte generiert wurden. Durch Expression transformierender Polypeptide von DNA-Viren können primäre Zellen in permanent wachsende Zellen überführt werden. Der Mechanismus der Transformation erfordert die Interaktion der virus-kodierten Genprodukte mit zellulären Proteinen, die an der Regulation der Zellteilung beteiligt sind (sog. Tumorsuppressorgenprodukte). Dadurch wird ein Haltepunkt des Zellzyklus (GO/G1 Phase) durchbrochen und die Zellen gehen in die S-Phase über, was zu einer unkontrollierten Proliferation der Zellen führt (Helt & Falloway, Carcinogenesis 24:159-169, 2003). Beispiele für solche transformierenden, virus-kodierten Genprodukte sind T-Antigen von Simian Virus 40 (SV40), E6/E7 des humanen Papillomavirus (HPV) oder E1A/E1B von Adenovirus. Diese viralen Proteine sind vergleichbar in ihrem Mechanismus der Transformation: T-Antigen, E6 und E1A inaktivieren Genprodukte der zellulären Retinoblastom-Proteinfamilie (pRb), und T-Antigen, E7 und E1B inaktivieren den zellulären Tumorsuppressor p53 (zusammengefasst in: zur Hausen, J. Natl. Cacer Inst, 92, 690-698, 2000; Ludlow, FASEB J. 7: 866-871, 1993; Moran, FASEB J. 7, 880-885, 1993). Die so transformierten Zelllinien werden dadurch dauerhaft kultivierbar und sind besonders geeignet für biotechnologische Produktionszwecke.

Nur ganz wenige menschliche Zelltypen wurden bisher mit der E1-Genregion des Adenovirus transformiert. Dazu gehörten neuronale Zellen in humanem embryonalem Nierengewebe (HEK293) (Graham et al., J. Gen. Virol. 36:59-74, 1977), humane embryonale Retinazellen (EP 833 934 B1) und humane Amniozyten (EP 1 230 354 B1).

Das beschriebene Verfahren zur Herstellung von Produktionszelllinien auf der Basis vorhandener immortalisierter bzw. transformierter Zelllinien hat in verschiedener Hinsicht Nachteile. Der Zusatz von Antibiotika, Chemotherapeutika oder anderen Selektionsreagenzien zum Kulturmedium während der Selektionsphase und unter Umständen auch in der Expansionsphase vor der Produktion erhöht die Kosten des Produktionsverfahrens. Durch aufwändige Analysen im Produktionsprozess sowie des Endproduktes muss außerdem sichergestellt und nachgewiesen werden, dass das gereinigte und konfektionierte Endprodukt keine Reste der Selektionsreagenzien enthält. Dies bedeutet zusätzliche Ansprüche an die Qualitätskontrolle, um die Sicherheit des therapeutischen Präparats zu gewährleisten. Manche der eingesetzten Selektionsreagenzien haben zudem Einfluss auf die Qualität bzw. klonale Stabilität der Produktionszelle. Der kontinuierliche Zusatz von Methotrexat als Selektionsreagenz in der Kultur beispielsweise hält zwar bei manchen Produktionszelllinien den Eindruck des "stabilen" Phänotyps der Zelle im Hinblick auf ihre Produktivität aufrecht. Genetische Studien haben aber gezeigt, dass die Anwesenheit dieses Reagenz die cytogenetische Heterogenität der Zellen fördert, was insbesondere im Hinblick auf die regulatorischen Erfordernisse des Zulassungsprozesses unerwünscht ist (Wurm et al., Dev. Biol. Stand. 76:69-82, 1992; Kim und Lee, Biotechnol. Bioeng. 64:741-749, 1999). Ein weiterer Nachteil der klassischen Methode zur Generierung von Produktionszelllinien kann darin gesehen werden, dass nur eine sehr begrenzte Auswahl an permanenten Ausgangszelllinien, auf denen das Herstellungsverfahren basiert (z.B. CHO, BHK, HEK293), vorhanden ist. Diese Zellen sind alle vor ihrer Entwicklung zu Produktionszelllinien schon über viele Jahre in zahllosen Passagen kultiviert und gelagert worden. Diese Vorgänge bergen immer das Risiko, zusätzlich zu den für die initiale Immortalisierung notwendigen genetischen Veränderungen zahlreiche weitere chromosomale Aberrationen und genetische Mutationen anzuhäufen, die sie der "natürlichen" tierischen bzw. menschlichen Ausgangszelle immer unähnlicher machen. Mögliche Konsequenzen dieser Veränderungen auf sicherheitsrelevante und/oder produktionstechnische Aspekte der gentechnischen Herstellung von rekombinanten Proteinen sind bisher nicht eingehend untersucht worden.

Daher liegt der Erfindung die Aufgabe zu Grunde, ein einfacheres, kostengünstigeres und unter Toxizitätsaspekten sichereres Verfahren zur Herstellung von permanenten humanen Zelllinien für die Produktion von rekombinanten Polypeptiden bereitzustellen.

Die Aufgabe wird durch den in den Patentansprüchen definierten Gegenstand gelöst.

Die nachfolgenden Figuren erläutern die Erfindung.
Fig. 1 zeigt schematisch den Aufbau des Plasmids pGS119 zur Expression der E1-Genfunktionen und des viralen Strukturproteins pIX von Adenovirus Serotyp 5.
Fig. 2 zeigt schematisch den Aufbau des Plasmids pGS116 zur Expression einer cDNA des humanen Alpha1-Antitrypsins (hAAT) unter der Kontrolle des Cytomegalie-Virus (CMV)-Promotors.
Fig. 3 zeigt das Ergebnis eines Western Blots zur Expression der E1A- und E1B-Proteine von Ad5 in sieben verschiedenen permanenten Amniozyten-basierten Produktionszelllinien für humanes Alphal-Antitrypsin.
Fig. 4 zeigt das Ergebnis eines Western Blots zur Expression von humanem Alphal-Antitrypsin (hAAT) in sieben verschiedenen permanenten Amniozyten-basierten Produktionszelllinien. A, aus den Zellen in den Kulturüberstand sezerniertes hAAT; B, intrazelluläres hAAT.
Fig. 5 zeigt schematisch die Menge des von ausgewählten Produktionszelllinien exprimierten humanen Alphal-Antitrypsins (hAAT) im Kulturüberstand (ELISA, Enzyme-linked Immunosorbent Assay). A, Menge an hAAT in Passage 5 der einzelnen Zellklone; B, Stabilität der Expression über mehrere Passagen in ausgewählten Zellklonen.
Fig. 6 zeigt das Ergebnis eines Western Blots zur Glykosylierung von humanem Alpha1-Antitrypsin (hAAT) aus Amniozyten-basierten Produktionszelllinien

Unter dem Begriff "Amniozyten" wie hier verwendet versteht man im weiteren Sinne alle Zellen, die im Fruchtwasser vorhanden sind und z.B. durch Fruchtwasserpunktion gewonnen werden können. Sie stammen entweder vom Amnion oder von fetalem Gewebe, das im Kontakt mit der Amnionflüssigkeit ist. Es wurden drei Hauptklassen von Amniozyten beschrieben, die auf Grund morphologischer Kriterien unterschieden werden: Fibroblastenartige Zellen (F-Zellen), epitheloide Zellen (E-Zellen) und Amnionflüssigkeitszellen (Amniotic Fluid Cells, AF-Zellen) (Hohn et al., Pediat. Res. 8:746-754, 1974). AF-Zellen sind der vorherrschende Zelltyp. Als Zellquelle für Amniozyten können Zellen aus Fruchtwasserpunktionen (Amniozentesen) dienen, die z.B. für eine zytogenetische Diagnostik durchgeführt werden. Als Zellquelle für Amniozyten können auch Zellen aus Fruchtwasserentnahmen dienen, die bei überdurchschnittlich großen Fruchtwassermengen während der Schwangerschaft (Polyhydramnion, Polyhydramnie) vorgenommen werden.

Unter dem Begriff "Expressionskassette" wird insbesondere ein Nukleinsäuremolekül bzw. ein Bereich eines Nukleinsäuremoleküls verstanden, das ein vor der codierenden Region liegendes regulatorisches Element oder einen Promotor, eine codierende Region bzw. einen offenen Leserahmen, sowie ein hinter der codierenden Region liegendes transkriptionelles Terminationselement enthält. Das vor der codierenden Region liegende regulatorische Element bzw. der Promotor kann ein konstitutiver, d.h. permanent die Transkription aktivierender Promotor (z.B. CMV-Promotor) oder ein regulierbarer, d.h. an- und/oder abschaltbarer Promotor (z.B. Tetrazyklin-regulierbarer Promotor) sein. Die codierende Region der Expressionskassette kann ein durchgehender offener Leserahmen wie bei einer cDNA mit einem Startcodon am 5'-Ende und einem Stoppcodon am 3'-Ende sein. Die codierende Region kann aus einer genomischen oder einer neu kombinierten Anordnung von codierenden Exons und dazwischenliegenden, nicht-codierenden Introns bestehen. Die codierende Region der Expressionskassette kann aber auch aus mehreren offenen Leserahmen bestehen, die durch sog. IRES (Internal Ribosome Entry Sites) voneinander getrennt sind.

Unter dem Begriff permanente Zelllinien" wie hier verwendet versteht man Zellen, die derart genetisch verändert sind, dass sie unter geeigneten Kulturbedingungen permanent in Zellkultur weiterwachsen können. Solche Zellen werden auch immortalisierte Zellen genannt.

Unter dem Begriff "Polypeptid" oder "rekombinantes Polypeptid" wie hier verwendet werden Peptide aus mindestens 2 Aminosäuren verstanden. Das Polypeptid kann ko- und/oder posttranslational modifiziert werden, z.B. durch das Anhängen von Zuckerresten oder durch Modifikation von Aminosäureresten. Das Polypeptid kann linear, zirkulär oder verzweigt sein. Ferner kann das Polypeptid aus mehr als einer Aminosäurekette bestehen, wobei die Ketten durch intra- und/oder intermolekulare Bindungen mehr oder weniger komplexe Raumstrukturen einnehmen können (z.B. Sekundär-, Tertiär-, Quartärstruktur). Besteht das Polypeptid aus einer Aminosäurekette, kann diese auch durch intramolekulare Bindungen mehr oder weniger komplexe Raumstrukturen einnehmen. Die Polypeptide können pharmakologisch oder immunologisch aktive Polypeptide oder für diagnostische Zwecke verwendete Polypeptide sein.

Unter dem Begriff "primäre Zellen" wie hier verwendet versteht man Zellen, die durch direkte Entnahme aus einem Organismus oder einem Gewebe erhalten und in Kultur genommen wurden. Primäre Zellen besitzen nur eine sehr begrenzte Lebensdauer. Primäre Zellen können z.B. Amniozyten, neuronale Zellen oder Retinazellen sein.

Unter dem Begriff "Produktionszelllinien" wie hier verwendet versteht man permanente Zelllinien, die durch Einbringen eines Transgens, welches das zu produzierende gewünschte Polypeptid codiert, genetisch verändert wurden.

Unter dem Begriff "Selektionsmarker" wie hier verwendet versteht man einen genetischen Marker, der der Zelle unter Selektionsbedingungen einen Wachstumsvorteil verschafft. Solche Selektionsbedingungen können beispielsweise die Gegenwart eines Chemotherapeutikums oder eines Antibiotikums oder einer anderen cytotoxischen oder wachstumsbeeinträchtigenden Substanz im Medium sein. Häufig benutzte Selektionsmarker sind das Neomycin-Resistenzgen, das Hygromycin-Resistenzgen und die Dihydrofolat-Reduktase.

Unter dem Begriff "Transfektion" wie hier verwendet wird jedes Verfahren verstanden, das sich zum Einbringen der genannten Nukleinsäure(n) in die Zellen eignet. Als Beispiele sind die klassische Kalziumphosphat-Methode, Elektroporation, liposomale Systeme jeglicher Art und Kombinationen dieser Verfahren zu nennen.

Unter dem Begriff "Transgen" wie hier verwendet versteht man die ein rekombinantes Polypeptid codierende Nukleinsäuresequenz.

Ein Gegenstand der vorliegenden Erfindung betrifft ein Verfahren zur Herstellung einer permanenten humanen Zelllinie, wobei isolierte primäre humane Zellen mit einem Nukleinsäuremolekül oder mindestens zwei verschiedenen Nukleinsäuremolekülen transfiziert werden, und falls ein Nukleinsäuremolekül transfiziert wird, das Nukleinsäuremolekül eine Sequenz aufweist, die die Expression von mindestens einem Zell-transformierenden Faktor und die Expression eines rekombinanten Polypeptids erlaubt, und falls mindestens zwei verschiedene Nukleinsäuremoleküle transfiziert werden, ein erstes Nukleinsäuremolekül eine Sequenz aufweist, die die Expression von mindestens einem zell-transformierenden Faktor erlaubt, und ein zweites Nukleinsäuremolekül eine Sequenz aufweist, die die Expression mindestens eines rekombinanten Polypeptids erlaubt.

Die für das Verfahren der vorliegenden Erfindung verwendeten primären humanen Zellen werden durch direkte Entnahme aus dem Organismus oder einem dem Organismus entnommenen Gewebe erhalten und in Kultur genommen. Bevorzugt sind solche primären humanen Zellen, die durch Expression mit Zell-transformierenden Faktoren gut in permanente humane Zelllinien umgewandelt werden können, insbesondere Amniozyten.

Zell-transformierende Faktoren können T-Antigen von SV40 (Genbank Acc. Nr. J02400), E6- und E7-Genprodukt von HPV (z.B. HPV16, Genbank Acc. Nr. K02718) und E1A- und E1B-Genprodukte von humanen Adenoviren (z.B. humanes Adenovirus Serotyp 5, Genbank Acc. Nr. X02996). Werden für das erfindungsgemäße Verfahren Nukleinsäuresequenzen für die E6- und E7-Genprodukte verwendet, müssen die primären humanen Zellen mit den Sequenzen für beide Genprodukte transfiziert werden. Werden für das erfindungsgemäße Verfahren Nukleinsäuresequenzen für die E1A- und E1B-Genprodukte verwendet, müssen die primären humanen Zellen mit den Sequenzen für beide Genprodukte transfiziert werden. Bei der Expression durch ein natürlicherweise vorliegendes HPV können E6 und E7 von einem RNA-Transkript exprimiert werden. Gleiches gilt für die Expression von E1A und E1B eines natürlicherweise vorliegenden Adenovirus. Die zell-transformierenden Faktoren wie z.B. die adenovirale E1-Genfunktion bewirken die Immortalisierung oder Transformation und somit die dauerhafte Kultivierbarkeit der Zellen. Nicht transfizierte primäre humane Zellen sterben natürlicherweise in der Kultur spätestens nach einigen Wochen ab, während die transfizierten Zellen auf Grund der immortalisierenden Wirkung z.B. der E1-Genprodukte zu phänotypisch erkennbaren Zellkolonien heranwachsen. Der für die Immortalisierung der Zellen essentielle Transfer der zell-transformierenden Faktoren übernimmt damit im erfindungsgemäßen Verfahren die Funktion eines Selektionsmarkers, der ohne zusätzlichen äußeren chemischen Selektionsdruck die Isolierung von Zelllinien erlaubt, die ein oder mehrere verschiedene rekombinante Polypeptide exprimieren. Die vorliegende Erfindung betrifft daher insbesondere ein Verfahren zur Herstellung einer permanenten humanen Zellinie ohne Transfektion eines Selektionsmarkers.

Die Nukleinsäuresequenzen für die Expression der Zell-transformierenden Faktoren liegen in Form einer Expressionskassette vor.

Die Expressionskassetten können homologe Promotoren bzw. transkriptionelle Terminationssequenzen enthalten, z.B. den natürlichen E1A-Promotor und die natürliche E1A-Polyadenylierungsstelle für die Expression der adenoviralen E1A-Genfunktion. Dies kann erreicht werden, indem die für die Transfektion benutzten Nukleinsäuremoleküle Fragemente des jeweiligen viralen Genoms, z.B. des adenoviralen Genoms, mit den genannten Genfunktionen, z.B. E1A, E1B, enthalten. Die Expressionskassetten für die zell-transformierenden Faktoren können aber auch heterologe, nicht natürlicherweise mit der verwendeten codierenden Region vorkommende Promotoren oder transkriptionelle Terminationssequenzen enthalten. Als heterologe Promotoren können z.B. CMV- (Cytomegalievirus-) Promotor (Makrides, 9-26 in: Makrides (Hrsg.), Gene Transfer and Expression in Mammalian Cells, Elsevier, Amsterdam, 2003), EF-1α-Promotor (Kim et al., Gene 91:217-223, 1990), CAG-Promotor (ein Hybridpromotor aus dem Immediate Early-Enhancer des humanen Cytomegalievirus und einem modifizierten Huhn β-Aktin Promotor mit erstem Intron) (Niwa et al., Gene 108:193-199, 1991), humaner oder muriner pgk- (Phosphoglyceratkinase-)Promotor (Adra et al., Gene 60:65-74, 1987), RSV- (Rous Sarkoma Virus-) Promotor (Makrides, 9-26 in: Makrides (Hrsg.), Gene Transfer and Expression in Mammalian Cells, Elsevier, Amsterdam, 2003) oder SV40- (Simian Virus 40-) Promotor (Makrides, 9-26 in: Makrides (Hrsg.), Gene Transfer and Expression in Mammalian Cells, Elsevier, Amsterdam, 2003) dienen. Als Polyadenylierungsstellen können z.B. die Polyadenylierungssequenzen des SV40 Large T-Antigens (Genbank Acc. Nr. J02400) oder des humanen G-CSF- (Granulozyten-Kolonie-stimulierender Faktor, granulocyte colony stimulating factor) Gens (Mizushima und Nagata, Nucl. Acids Res. 18:5322, 1990) dienen. Ebenso können manche der Expressionskassetten für die transformierenden Faktoren homologe Regulationselemente und andere heterologe Regulationselemente enthalten. So kann z.B. die EIA-Expressionskassette einen heterologen Promotor, die E1B-Expressionskassette einen homologen Promotor oder umgekehrt oder beide Expressionskassetten einen heterologen Promotor aufweisen.

Auch die Nukleinsäuresequenzen für die Expression des mindestens einen rekombinanten Polypeptids liegen in mindestens einer Expressionskassette vor, wobei die Art und die Auswahl der Promotoren und die transkriptionellen Terminationssequenzen den oben für die Zell-transformierenden Faktoren genannten entsprechen.

Die Nukleinsäuresequenzen der zell-transformierenden Faktoren und des rekombinanten Polypeptids einschließlich der jeweiligen Regulationselemente können auf einem oder mehreren verschiedenen Nukleinsäuremolekülen liegen. In einer bevorzugten Ausführungsform der vorliegenden Erfindung liegen die Sequenzen der zell-transformierenden Faktoren einschließlich der jeweiligen Regulationselemente, z.B. die E6- und E7-Genfunktionen oder die E1A- und E1B-Genfunktionen, oder der zell-transformierende Faktor, z.B. das T-Antigen von SV40, auf einem Nukleinsäuremolekül und die Sequenzen des rekombinanten Polypeptids einschließlich der Regulationselemente auf einem vom ersten Nukleinsäuremolekül verschiedenen zweiten Nukleinsäuremolekül. In einer weiteren bevorzugten Ausführungsform liegen auch die Sequenzen der transformierenden Faktoren E6 und E7 bzw. E1A und E1B einschließlich der jeweiligen Regulationselemente auf verschiedenen Nukleinsäuremolekülen. Die Sequenz des rekombinanten Polypeptids einschließlich der Regulationselemente kann in diesem Fall auf einem vom ersten und zweiten Nukleinsäuremolekül verschiedenen dritten Nukleinsäuremolekül oder auf dem ersten oder zweiten Nukleinsäuremolekül, enthaltend die Sequenz für einen der Zell-transformierenden Faktoren liegen.

In einer bevorzugten Ausführungsform werden die primären humanen Zellen mit einem ersten Nukleinsäuremolekül enthaltend die Expressionskassette der E1A und E1B-Genfunktionen und einem vom ersten Nukleinsäuremolekül verschiedenen zweiten Nukleinsäuremolekül transfiziert, das die Expressionskassette des rekombinanten Polypeptids aufweist. In dieser Ausführungsform enthält das erste Nukleinsäuremolekül die E1A-Expressionskassette enthaltend einen heterologen Promotor und das homologe transkriptionelle Terminationselement, und die E1B-Transkriptionseinheit den homologem Promotor und das homologe transkriptionelle Terminationselement. In einer besonders bevorzugten Ausführungsform enthält das erste Nukleinsäuremolekül die Adenovirus Serotyp 5 Nukleotidsequenz von Nukleotid 505 bis Nukleotid 4079. In einer weiteren besonders bevorzugten Ausführungsform enthält das erste Nukleinsäuremolekül die Adenovirus Serotyp 5 Nukleotidsequenz von Nukleotid 505 bis Nukleotid 3522. In einer weiteren besonders bevorzugten Ausführungsform enthält das erste Nukleinsäuremolekül die Adenovirus Serotyp 5 Nukleotidsequenz von Nukleotid 1 bis Nukleotid 4344, die der adenoviralen DNA in HEK293-Zellen entspricht (Louis et al., Virology 233:423-429, 1997).

Das Vorhandensein des E1B-eigenen Promotors und der E1B-eigenen Polyadenylierungssequenz (Nukl. 4037-4070) sollten zu einer optimalen Transkription und Expression von E1B bei dieser Ausführungsform führen. Ein weiterer Vorteil liegt darin, dass der Sequenzbereich zwischen dem Stoppcodon von E1B und der Polyadenylierungsstelle von E1B die adenovirale pIX-Genfunktion enthält. Das pIX-Polypeptid, ein virales Strukturprotein, wirkt als Transkriptionsaktivator auf verschiedene virale und zelluläre Promotoren wie z.B. den Thymidinkinase- und den Beta-Globin-Promotor. Die transkriptionsaktivierende Wirkung des zusätzlich in der Zelle exprimierten pIX-Polypeptids kann in den erfindungsgemäßen Produktionszelllinien zu einer Erhöhung der Expressionsraten des rekombinanten Polypeptids führen, falls die codierende Sequenz des rekombinanten Polypeptids unter der Kontrolle eines der zuvor genannten Promotoren steht.

Die rekombinanten Polypetide können therapeutische Proteine wie z.B. humanes Alpha 1-Antitrypsin oder Wachstumsfaktoren wie Erythropoietin oder Interleukin-2 sein. Humanes Alpha 1-Antitrypsin (hAAT) ist ein Proteinase-Inhibitor, der Elastase und andere Proteinasen inhibiert und bei erblichem hAAT-Mangel, der zu schweren Lungen- und Leberschädigungen führt, therapeutisch wirksam ist. Bei Erythropoietin handelt es sich um einen wichtigen Wachstumsfaktor für Erythrozyten (rote Blutkörperchen), der bei Anämie sowie bei Transplantationspatienten blutbildende Wirkung hat. Interleukin-2 (Il-2) zählt zu den zellulären Botenstoffen des Immunsystems und hat große Bedeutung bei der Aktivierung der zellulären Immunantwort beispielsweise bei Tumorerkrankungen. Zu den therapeutisch wirksamen Polypeptiden zählen auch Blutgerinnungsfaktoren wie z.B. Faktor VIII und Faktor IX, die bei Hämophilie-Patienten mit Gerinnungsstörungen eingesetzt werden. Das rekombinante Polypeptid des erfindungsgemäßen Verfahrens kann ein Hormon sein. Biotechnologisch hergestellte Hormone werden in der Substitutionstherapie bei Patienten mit hormonellen Störungen verwendet. Beispiele sind das blutzuckersenkende Hormon Insulin, auf das viele Diabetes mellitus-Patienten angewiesen sind, Somatotropin (Wachstumshormon) für die Behandlung von Zwergwüchsigkeit, und gonadotrope Faktoren wie Follikelstimulierendes Hormon (FSH) oder Luteinisierendes Hormon (LH) für die Behandlung von Fertilitätsstörungen.

Das rekombinante Polypetid kann ein rekombinanter Antikörper sein, der zu therapeutischen oder diagnostischen Zwecken eingesetzt werden kann. Antikörper gegen den Tumor-Nekrose-Faktor alpha (TNF-α) werden bei Patienten mit rheumatoider Arthritis, Antikörper gegen den zellulären Rezeptor des Epidermalen Wachstumsfaktors (EGFR) bei Krebspatienten eingesetzt. Für diagnostische Zwecke eingesetzte Antikörper können beispielsweise Bestandteile von kommerziellen Diagnose-Kits sein, die auf Verfahren wie Enzym-gekoppelte Immun-Adsortions-Tests (Enzyme-linked Immuno Sorbent Assay, ELISA) oder radioaktiven Immun-Adsorptions-Tests (Radio Immuno Sorbent Assay, RIA) beruhen. Die Antikörper dienen in diesen Testverfahren zum Nachweis der Antigene von Infektionserregern, wie beispielsweise dem humanen Hepatitis B-Virus.

Antikörper oder Immunglobuline (Ig) setzen sich aus einer schweren und einer leichten Kette zusammen, die jeweils aus variablen und konstanten Regionen oder Domänen bestehen. Die Nukleinsäuresequenzen der transfizierten Nukleinsäuremoleküle zur Expression eines Antikörpers können zwei getrennte Expressionskassetten enthalten, von denen die eine die leichte Kette, die andere die schwere Kette des Immunglobulinmoleküls codiert. Nach Expression beider Ketten in der erfindungsgemäßen Zelle lagern sich diese zum aktiven Antikörpermolekül zusammen. Die Expressionskassetten der beiden Ketten können dabei auf getrennten oder auf demselben Nukleinsäuremolekül liegen. Die codierenden Sequenzen für die leichte und die schwere Kette können aber auch innerhalb derselben Expressionskassette liegen und durch eine IRES-Sequenz (internal ribosome entry site, interne Ribosomenbindungsstelle) getrennt werden, die eine Expression sowohl der schweren als auch der leichten Kette gewährleistet. Die codierenden Sequenzen für die leichte und die schwere Kette können prinzipiell auch innerhalb derselben Expressionskassette liegen und durch eine Sequenz getrennt werden, die eine enzymatische Spaltstelle für eine Proteinase (z.B. Thrombin) codiert, welche dann in der Zelle gleichzeitig exprimiert wird und das Vorläufer-Polypeptid aus Leicht- und Schwerkettensequenz in aktive leichte und schwere Kette spaltet.

Rekombinante Antikörper, die von der Nukleinsäuresequenz in der erfindungsgemäßen Zelle codiert werden, können auch aus Fragmenten eines Antikörpers bestehen anstelle der kompletten leichten und schweren Kette. Sogenannte Einzelketten-Antikörper (scFv, single chain variable fragments) bestehen aus den variable Domänen einer schweren und einer leichten Kette, die durch eine Aminosäuresequenz (einen sog. Linker) verbunden werden, die eine freie Beweglichkeit beider Domänen gewährleistet. Durch intramolekulare Zusammenlagerung beider Domänen ensteht eine antigenbindende Struktur, die dem variablen Bereich eines Immunglobulinmoleküls entspricht. Bispezifische Einzelketten-Antikörper (bis-scFv) bestehen aus zwei solchen Einzelketten-Anordnungen aus den variablen Domänen einer schweren und einer leichten Kette, die ihrerseits wiederum durch eine verbindende Sequenz zusammenhängen und gegeneinander beweglich sind; solche Moleküle können an zwei antigene Bindungsstellen (Epitope) gleichzeitig binden und dadurch zwei molekulare Strukturen nicht-kovalent verbinden. Bispezifische Diabodies bestehen aus zwei getrennt exprimierten Einzelketten, die jeweils variable Domänen einer leichten und einer schweren Kette, getrennt nur durch einen sehr kurzen oder ganz ohne Linker, enthalten. Der kurze oder fehlende Linker verhindert die intramolekulare Zusammenlagerung, durch intermolekulare Zusammenlagerung einer variablen schweren und leichten Domäne entsteht wiederum ein aktives Molekül mit zwei Bindungsvalenzen.

Die von den im vorliegenden Verfahren transfizierten Nukleinsäuremolekülen codierten rekombinanten Polypetide können virale, bakterielle oder parasitäre Proteine sein, die für eine Verwendung als prophylaktische oder therapeutische Impfstoffe (Vakzine) produziert werden sollen. Dabei kann es sich sowohl um Strukturpolypeptide als auch um regulatorische oder enzymatisch aktive Polypeptide von Viren, Bakterien oder Parasiten handeln. Virale Protein können z.B. das Hepatitis B-Virus Oberflächen-Antigen (HBV Surface Antigen) oder das Strukturprotein L1 von humanen Papillomviren sein. Bakterielle Proteine, die nach Expression in Produktionszelllinien für die Impfstoffherstellung in Frage kommen, sind z.B. Enterotoxin-Untereinheiten von enterotoxinogenen Escherichia coli (ETFC) oder Transferrin-bindende Proteine (Transferrin binding proteins, Tbp A und B) von Neisseria gonorrhoeae. Polypeptide von Parasiten, die von den im vorliegenden Verfahren transfizierten Nukleinsäuremolekülen codiert werden könnten, sind z.B. das Merozoiten-Oberflächenprotein (Merozoite Surface Protein, MSP) des Malariaerregers Plasmodium falciparum oder Glutathion S-transferase (GST) von Schistosoma japonicum.

Die von den im vorliegenden Verfahren transfizierten Nukleinsäuremolekülen codierten rekombinanten Polypetide können ein oder mehrere virale Polypeptide sein, die als Replikationsfaktoren wirken und eine episomale, d.h von Chromosomen unabhängige Replikation von später durch Transfektion in die Zelllinie eingebrachten Nukleinsäuremolekülen in der humanen Zelllinie erlauben. Die später in die Zelllinie durch Transfektion eingebrachten Nukleinsäuremoleküle enthalten ein als Replikationsursprung (ori, "origin of replication") bezeichnetes genetisches Element, an welches das bzw. die als Replikationsfaktoren wirkenden Polypeptide binden und dadurch die Replikation des episomalen Nukleinsäuremoleküls initiieren. Die episomale Replikation von Nukleinsäuremolekülen, insbesondere von Plasmid-DNA, in Zellen bewirkt eine starke Vermehrung der Kopienzahl der transferierten Nukleinsäuremoleküle und dadurch eine Erhöhung der Expression eines auf diesem Molekül codierten rekombinanten Polypeptids sowie dessen Erhalt über viele Zellteilungen hinweg. Ein solcher viraler Replikationsfaktor ist z.B. das T-Antigen des Simian Virus 40 (SV40), das nach Bindung an eine als SV40-Replikationsursprung (SV40 ori, origin of replication) bezeichnete Sequenz auf dem Nukleinsäuremolekül, z.B. der Plasmid-DNA, dessen Replikation initiiert. Das Ebstein-Barr-Virus Protein EBNA-1 (Ebstein Barr Virus Nuclear Antigen-1) erkennt einen als ori-P bezeichneten Replikationsursprung und katalysiert die extrachromosomale Replikation des ori-P tragenden Nukleinsäuremoleküls.

Die von den im vorliegenden Verfahren transfizierten Nukleinsäuremolekülen codierten rekombinanten Polypetide können außerdem virale Proteine sein, die in Zelllinien eine Produktion von rekombinanten viralen Gentransfer-Vektoren erlauben. Diese viralen auch Komplementationsfaktoren genannten Proteine werden in der Zelllinie exprimiert und sind die für die Produktion der Gentransfer-Vektoren notwendigen enzymatischen oder strukturellen Komponenten, die nicht auf dem Nukleinsäuremolekül des Gentransfer-Vektors codiert werden. In solchen Gentransfer-Vektoren sind in der Regel aus Sicherheitsgründen bestimmte virale Genfunktionen deletiert. Zu den Gentransfer-Vektoren, deren Komplementationsfaktoren durch mit dem beschriebenen Verfahren eingebrachte Transgene codiert werden können, gehören beispielsweise Vektoren, die auf Adenovirus, Adenovirus-Assozüertem Virus (AAV) oder Herpesvirus basieren. Die in der Zelllinie exprimierten Komplementationsfaktoren können auch deletierte oder rekombinante Viren bei ihrer Produktion komplementieren, die kein zu transferierendes Gen enthalten und damit nicht als Gentransfer-Vektoren fungieren, sondern z.B. als Impfstoff verwendet werden.

Im Falle von Adenovirus-basierten Gentransfer-Vektoren können in der erfindungsgemäßen Zelle beispielsweise die Genfunktionen pIX, E2, E3, und/oder E4 exprimiert werden. Die Deletion der entsprechenden Genfunktion im Genom des Vektors erhöht die Sicherheit der Vektoren und vergrößert dessen Kapazität zur Aufnahme fremder Nukleinsäuresequenzen. Die Genfunktion pIX ist ein adenovirales Strukturprotein, d.h. ein Bestandteil des Viruskapsids. Die viralen Genfunktionen E2, E3 und E4 codieren in der Frühphase der Virusvermehrung exprimierte regulatorische Polypeptide. Adenovirale Vektoren der ersten Generation sind lediglich E1- und/oder E3-deletiert. Adenoviralen Vektoren der zweiten Generation fehlen zusätzlich die Genfunktionen E2 und/oder E4. Zelllinien, die sämtliche viralen Genfunktionen komplemetieren, wären prinzipiell in der Lage, die als "Gutless" Vektoren oder adenovirale Vektoren hoher Kapazität bezeichnete Generation adenoviraler Gentransfer-Vektoren zu komplementieren, die selbst keine codierenden Genfunktionen mehr besitzen, sondern nur noch die als terminale Endwiederholungen (ITRs, Inverted Terminal Repeats) bezeichneten Sequenzen für die Replikation der Vektor-Nukleinsäure.

Die mit dem vorliegenden Verfahren in die primäre humane Zelle transfizierte Nukleinsäure kann ferner ein Rezeptorpolypeptid codieren, das insbesondere auf der Oberfläche der Zelle lokalisiert ist und für die Infektion der Zelle durch ein Virus bzw. die Transduktion der Zelle durch einen viralen Gentransfer-Vektor verantwortlich ist. Als viraler Rezeptor für den initialen Schritt der Infektion von Zellen mit dem Adenovirus Serotyp 2 oder 5, von denen die meisten konventionellen adenoviralen Vektoren abgeleitet sind, wurde der sog. Coxsackie- und Adenovirus-Rezeptor, CAR, identifiziert (Bergelson et al., Science 275:1320-1323, 1997). Die hinreichende Expression von CAR auf der Oberfläche ist eine Voraussetzung dafür, dass sich eine Zelle als Produktionszelle für adenovirale Gentransfer-Vektoren eignet. In einer bevorzugten Ausführungsform ist das rekombinante Polypeptid der Coxsackie- und Adenovirus-Rezeptor (CAR). Die Überexpression des Rezeptorpolypeptids kann die Infizierbarkeit und damit die Produktionseffizienz dieser Zellen hinsichtlich adenoviraler Vektoren deutlich verbessern. Ferner kann das Nukleinsäuremolekül außer CAR sekundäre Rezeptoren oder Internalisierungsrezeptoren codieren, wie z.B. bestimmte Integrine, die die Aufnahme des Virus bzw. Gentransfer-Vektors in die Zelle vermitteln und deren zusätzliche Expression bei der Herstellung von Produktionszellen für adenovirale Vektoren sinnvoll sind.

Die im vorliegenden Verfahren zur Transfektion primärer humaner Zellen benutzten Nukleinsäuremoleküle, die das mindestens eine rekombinante Polypeptid codieren, oder die Nukleinsäuremoleküle, die die Zell-transformierenden Faktoren codieren, können "Matrix Attachment Regions" (MARs) enthalten. Diese auch als "Scaffold Attachment Regions" (SARs) bezeichneten genetischen Elemente spielen eine Rolle bei der Regulation der Genexpression, da sie mit Transkriptionseinheiten und regulatorischen Elementen im Genom assoziiert sind. MARs haben die Fähigkeit, in eine Zelle eingebrachte fremde Gene gegen eine transkriptionelle Stummschaltung oder Abschaltung der Transkription in der transfizierten Zelle zu schützen und dadurch ihre Expression zu verstärken (Girod und Mermod, S. 359-379, in S.C. Makrides (Hrg.) Gene Transfer and Expression in Mammalian Cells. 2003. Elsevier, Amsterdam).

Durch gleichzeitige Transfektion von primären humanen Zellen mit Nukleinsäuremolekülen enthaltend exprimierbare Nukleinsäuresequenzen für einen Zell-transformierenden Faktor bzw. zwei funktional miteinander verbundenen Faktoren und eine exprimierbare Nukleinsäuresequenz mindestens eines rekombinanten Polypeptids ist es den Erfindern gelungen, ohne Zusatz von Selektionsreagenzien permanente humane Zelllinien zur Produktion von rekombinanten Polypeptiden zu generieren. Die vorliegende Erfindung betrifft somit ferner ein Verfahren zur Herstellung einer permanenten humanen Zelllinie zur Expression mindestens eines gewünschten Polypeptids, vorzugsweise ohne Transfektion eines Selektionsmarkers. Die Erfindung betrifft ferner die mit dem erfindungsgemäßen Verfahren hergestellten permanenten humanen Zellen. Die Zellen können unter anderem zur Produktion therapeutischer Polypeptide, Blutgerinnungs- und Wachstumsfaktoren, Hormone und Antikörper sowie viraler, bakterieller oder parasitärer Polypeptide zur Verwendung als Impfstoff verwendet werden. Ferner sind die erfindungsgemäßen Zellen zur Produktion von diagnostisch relevanten Proteinen verwendbar, wie z.B. virale, bakterielle oder parasitäre Antigene oder entsprechende spezifische Antikörper. Ferner sind die erfindungsgemäßen Zellen zur Produktion von technisch oder industriell relevanten Proteinen verwendbar, z.B. von Enzymen zur Katalyse technischer Syntheseprozess oder zum Abbau von Schadstoffen. Die erfindungsgemäßen Zellen können ein oder auch mehrere verschiedene rekombinante Polypeptide exprimieren. Die Anzahl der exprimierbaren Polypeptide hängt davon ab, wie viele verschiedene Nukleinsäuresequenzen codierend für die rekombinanten Polypeptide mit dem erfindungsgemäßen Verfahren in die Zellen transfiziert werden.

Die Expressionsstärke eines Transgens in einer Produktionszelle hängt entscheidend von der genomischen Region ab, in die das Transgen nach dem Einbringen in die Zelle integriert. Während an manchen Stellen im zellulären Genom die Expression des Transgens nach relativ kurzer Zeit abgeschaltet wird, bleibt an anderen Stellen, sog. "Hot Spots" der Transkription, die Expressionskassette über lange Zeit mit hoher Effizienz transkriptionell aktiv. Die Aufrechterhaltung des transformierten Phänotyps in den erfindungsgemäß hergestellten permanenten Zellen erfordert die ständige Expression des Zell-transformierenden Faktors. In den permanenten humanen Zellen liegt also die Integrationstelle der entsprechenden Expressionskassette an einem "Hot Spot" der Transkripiton im zellulären Genom. Ein weiterer Aspekt der vorliegenden Erfindung betrifft somit ein Verfahren zur Herstellung einer permanenten humanen Zelle, wobei das Nukleinsäuremolekül, das die Sequenz für den oder die Zell-transformierenden Faktor(en) aufweist, ferner Erkennungssequenzen eines sequenzspezifischen Rekombinationsenzyms umfasst. In einem nachfolgenden Schritt kann in die permanenten humanen Zelllinien bei gleichzeitiger Expression der entsprechenden Rekombinase eine weitere Expressionskassette für ein Transgen eingebracht werden, die ebenfalls eine entsprechende Rekombinase-Erkennungssequenz oder Rekombinase-Erkennungssequenzen enthält. Dies führt zu einer Integration der Expressionskassette an den spezifischen "Hot Spot" und somit zu einer hohen, lang andauernden Expression des Transgens. Auf die beschriebene Weise erzeugte Produktionszelllinien mit nachgewiesen hoher Produktionseffizienz könnten verwendet werden, um sehr schnell effiziente Produktionszellen für ein anderes Transgen zu generieren. Ein weiterer Aspekt der vorliegenden Arbeit betrifft die Isolierung dieser spezifischen Hot Spots durch Sequenzanalyse der Integrationsstellen und deren Verwendung in Expressionsvektoren. Dies erlaubt die Isolierung bisher unbekannter Hot Spot Sequenzen aus humanen Zellen. Durch homologe Rekombination können in diese Sequenzen spezifisch Expressionkassetten integriert werden.

Ein weiterer Aspekt der vorliegenden Erfindung betrifft ein Verfahren zur Herstellung einer permanenten humanen Zelllinie, wobei das Nukleinsäuremolekül, das die Sequenz aufweist, die die Expression eines rekombinanten Polypeptids erlaubt, zusätzlich zur Expressionskassette des rekombinanten Polypeptids oder zur codierenden Sequenz des rekombinanten Polypeptids eine oder mehrere Rekombinase-Erkennungssequenzen aufweist. Z.B. können die Rekombinase-Erkennungssequenzen am 5'- und 3'- Ende der jeweiligen Sequenz liegen. Das rekombinante. Polypeptid kann in diesem Fall ein Reportergen sein, d.h. ein Gen, dessen Genprodukt leicht detektiert und quantitativ bestimmt werden kann. Nach Selektion einer stabilen Zelllinie mit starker Expression des Reporter-Proteins, d.h. des vom Reportergen codierten Genprodukts, kann in einem zweiten Schritt ein Nukleinsäuremolekül umfassend eine Expressionskassette für ein anderes rekombinantes Polxpeptid durch Transfektion in die Zelle eingebracht werden. Die Expressionskassette enthält ebenfalls Erkennungssequenzen des gleichen Rekombinationsenzyms. Nach kurzzeitiger Expression der Rekombinase wird durch Excision des ursprünglichen und Integration des neu in die Zelle transfizierten rekombinanten Polypeptids eine neue Produktionszelllinie mit gleichfalls hoher Produktionseffizienz entstehen. Dieses Verfahren verkürzt den zeitaufwendigen Prozess der Selektion gut exprimierender Produktionszelllinien. Beispiele für Rekombinasen und entsprechende Erkennungssequenzen sind: Cre-Rekombinase und loxP Erkennungssequenz aus dem Bakteriophage P1, Integrase und attB/attP Erkennungssequenz aus dem Bakteriophage φC31, oder Flp-Rekombinase und frt Erkennunsssequenz aus Hefen (Groth et al., PNAS 97: 5995-6000, 2000; Araki et al., Nucl. Acids Res. 25: 868-872, 1997; O'Gorman et al., Science 251: 1351-1355, 1991). Oft sind für eine effiziente Integration nicht die eigentlichen Erkennungssequenzen, sondern "pseudo" lox (Araki et al., Nucl. Acids Res. 25: 868-872, 1997) oder "pseudo" att (Thyagarajan et al., Mol. Cell Biol. 21: 3926-3934, 2001) von Vorteil.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### 1. Klonierungen

### a) Plasmid pSTK146

Plasmid pSTK146 wurde detailliert in EP 1 230 354 B1 beschrieben und umfasst den murinen Phosphoglyceratkinase (pgk) Promotor, Adenovirus Serotyp 5 (Ad5)-Sequenzen Nukleotid (nt.) 505 bis 3522 und das Spleiß- und Polyadenylierungssignal von SV40.

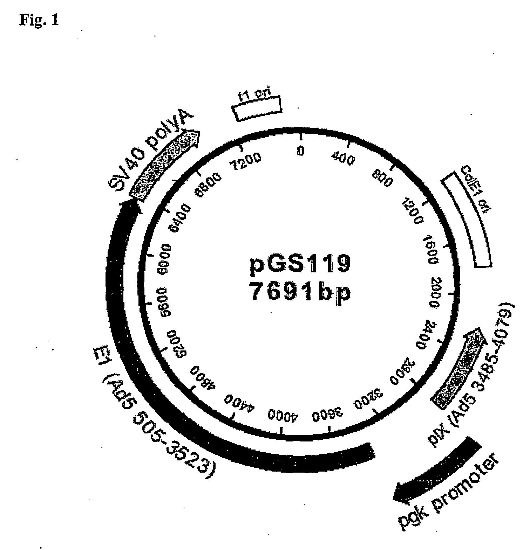

### b) Plasmid pGS119 (Fig.1)

Plasmid pGS 119 enthält den murinen pgk Promotor, Ad5-Sequenzen nt. 505-3522 enthaltend die gesamte E1-Region, das 3' Spleiß- und Polyadenylierungssignal von SV40 und die pIX-Region von Ad5 (nt. 3485-4079).
Die Ad5 pIX-Gensequenzen stammen aus Plasmid pXC1 (Microbix Biosystems Inc, Katalog-Nr. PD-01-03), welches Ad5-Sequenzen nt. 22-5790 enthält. Mit Hilfe dieses Plasmids und der Primer p9.3485-3504 (CTGGCTCGAGCTCTAGCGATGAAGATACAG; SEQ ID NO:1) und p9.4079-4060 (GCTGCTCGAGCACTTGCTTGATCCAAATCC; SEQ ID NO:2) wurden die Ad5-Gensequenzen nt. 3485-4079 durch Polymerase-Kettenreaktion (PCR) amplifiziert, mit XhoI gespalten (je eine XhoI-Schnittstelle lokalisiert in den Primern) und in die XhoI-Schnittstelle von pSTK146 eingeführt.

### c) Plasmid pGS122

Plasmid pGS122 enthält Ad5-Sequenzen bp 1-4344. In einem ersten Schritt wurden Ad5-Sequenzen nt. 356-3826 mittels SacII Verdau aus pXC1 (Microbix Biosystems Inc, Katalog-Nr. PD-01-03) isoliert und in die SacII-Schnittstelle von pSTK31 (enthält eine PmeI-Schnittstelle gefolgt von Ad5-Sequenzen bp 1-440, in pBluescript) eingeführt. Das so entstandene Plasmid pGS120 wurde mit pBstEII linearisiert und das BstEII-Fragment aus pXC1 enthaltend die Ad5-Sequenzen bp 1914-5185 eingeführt (pGS121). Zwei Oligonukleotide Ad5_4297-4344.PX (GCTGGGCGTGGTGCCTAAAAATGTCTTTCAGT AGCAAGCTGATTGCCAGTTTAAAC; SEQ ID NO:3) und Ad5_4344-4297 (TCGAGTTT AAACTGGCAATCAGCTTGCTACTGAAAGACATTTTTAGGCACCACGCCCAGCT; SEQ ID NO:4) wurden zum Doppelstrang hybridisiert. Plasmid pGS121 wurde mit AfeI und XhoI verdaut und das oben genannte Oligonukleotid eingefügt. Die Sequenz des Oligonukleotids wurde so gewählt, dass beim Einfügen in pGS121 am 5'-Ende die AfeI-Schnittestelle erhalten bleibt, und am 3'-Ende eine PmeI-Schnittstelle von der regenerierten XhoI-Schnittstelle gefolgt wird. Somit sind in pGS122 die Ad5-Sequenzen direkt von je einer PmeI-Schnittstelle flankiert.

### d) Plasmid pGS124

Plasmid pGS124 enthält den SV40-Promotor und die Gensequenz des SV40 T-Antigens. Durch Verdau von SV40 DNA mit BamPII und KpnI wurde ein 3-kb-Fragment aus dem SV40-Genom herausgeschnitten und in pBluescript, verdaut mit BamHI und KpnI, eingefügt. Plasmid pGS124 exprimiert somit das SV40 T-Antigen.

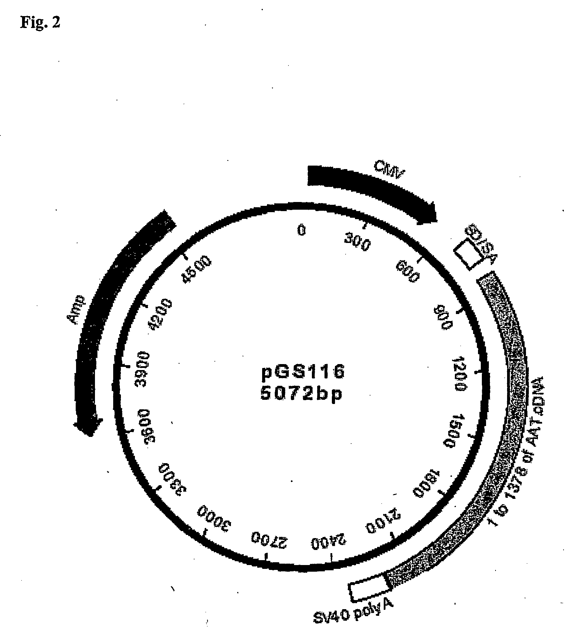

### e) Plasmide pGS116 (Fig. 2), pGS129, pGS131, pGS132, pGS133

Plasmide pGS116, pGS129, pGS131, pGS132 und pGS133 exprimieren das humane Alpha 1-Antitrypsin (hAAT) unter der Kontrolle verschiedener Promotoren.

Plasmid pGS116 (Fig. 2) enthält den frühen Promotor des humanen Cytomegalie-Virus (CMV), gefolgt von einer SV40-Spleißdonor/Spleißakzeptorstelle, der hAAT-cDNA und der SV40-Polyadenylierungsstelle (polyA). Die CMV- und SV40-Sequenzen stammen aus dem Plasmid pCMVβ (BD Clontech, Katalog-Nr. 6177-1). Das β-Galactosidasegen in pCMVβ wurde durch Verdau mit NotI und anschließender Auffüllreaktion des 5'-Überhangs entfernt und durch die hAAT-cDNA ersetzt, die durch EcoRI-Verdau des Plasmids Ad/PGK-hAAT (Kay et al., Hepatology 21:815-819, 1995) isoliert wurde.

Plasmid pGS129 enthält die oben beschriebene hAAT-cDNA unter der Kontrolle des humanen Promotors für den Elongationsfaktor EF-1α. Durch Verdau von pGS116 mit XhoI und EcoRI und anschließender Auffüllreaktion der 5'-Überhänge wurde der CMV-Promotor aus pGS116 entfernt und durch ein 1.3-kb-Fragment enthaltend den EF-1α Promotor ersetzt.

Plasmid pGS131 enthält die hAAT-cDNA unter Kontrolle eines Hybridpromotors bestehend aus dem CMV-Enhancer und dem Huhn β-Aktin-Promotor (CAG-Promotor). Durch Verdau von pGS116 mit XhoI und EcoRI und anschließender Auffüllreaktion der 5'-Überhänge wurde der CMV-Promotor durch den 1.1 kb großen CAG-Promotor ausgetauscht.

Plasmid pGS132 enthält die hAAT-cDNA unter Kontrolle des Rous Sarcomavirus (RSV)-Promotors. Der CAG-Promotor in pGS 116 wurde durch Restriktionsverdau mit XhoI und EcoRI und anschließender Auffüllreaktion der 5'-Überhänge entfernt und durch den 0.58 kb großen RSV-Promotor ersetzt.

In Plasmid pGS133 wird die Expression der hAAT-cDNA vom frühen Promotor von Simian Virus 40 (SV40) kontrolliert. Durch Verdau mit XhoI und EcoRI und anschließender Auffüllreaktion der 5'-Überhänge wurde aus pGS 116 der CMV-Promotor entfernt und durch den 0.23 kb großen SV40-Promotor ersetzt.

### f) Plasmid pGS126

Plasmid pGS126 enthält folgende Elemente: die E1-Expressionskassette bestehend aus pgk-Promotor, Ad5-Sequenzen nt. 505-3522, SV40 polyA, die pIX-Expressionskassette bestehend aus Ad5-Sequenzen nt. 3485-4079 und die hAAT-Expressionskassette bestehend aus CMV-Promotor, hAAT-cDNA, SV40 polyA. Durch Verdau von Plasmid pGS116 (siehe 1e) mit EcoRI und HindIII und anschließender Auffüllreaktion wurden die hAAT-Expressionskassette isoliert und in Plasmid pGS119 (siehe 1b), nach Verdau mit BamHI und Auffüllreaktion der 5'-Überhänge, einfügt. Plasmid pGS26 exprimiert somit die Ad5 E1- und pIX-Proteine und hAAT.

### g) Plasmid pGS123

Plasmid pGS123 enthält zusätzlich zu der hAAT-Expressionskassette eine Matrix-assozüerte Region (MAR). Dieses MAR lokalisiert in einem 2085 bp großen EcoRI/SpeI-Fragment aus der Intronregion des Genlokus für das humane AAT (GenBank Accession K02212). Dieses Fragment liegt kloniert in pBluscript (pGS58) vor und wurde in die EcoRI-Schnittstelle nach Auffüllreaktion der 5'-Überhänge von pGS116 (siehe 1e) eingeführt.

### h) Plasmid pGS127

Plasmid pGS127 enthält den CMV-Promotor, die cDNA für das humane Erythropoietin (Epo) und die SV40 polyA-Sequenz. Mit Hilfe eines NotI-Verdaus wurde das β-Galactosidasegen aus pCMVβ entfernt und durch die Epo-cDNA ersetzt.

### 2. Überprüfung der Konstrukte

### a) Sequenzanalyse

Die Vollständigkeit aller oben beschriebenen Plasmide wurde durch Restriktionsverdau überprüft. Weiterhin wurde die korrekte Sequenz der adenoviralen Fragmente in pGS119, pGS122 und pGS126 durch Sequenzanalyse bestätigt; es wurden keine Änderungen in der Sequenz im Vergleich zur Ad5 Wildtyp-Sequenz gefunden.

### b) Expression

Plasmide pGS116, pGS129, pGS131, pGS132, pGS133, pGS123 und pGS126 wurden in HEK293-Zellen transfiziert und die Expression und Sekretion von humanem Alpha1-Antitrypsin (hAAT) in den Kulturüberstand mittels ELISA (siehe Kapitel 6b) nachgewiesen.
Plasmide pGS119 und pGS122 wurden in HeLa-Zellen transfiziert und die Expression der E1A-Proteine mittels Western Blot unter Verwendung eines monoklonalen Antikörpers (siehe Kapitel 6a) analysiert.
Plasmid pGS124 wurde in HeLa- und HEK293-Zellen transfiziert und die Expression des T-Antigens mittels Western Blots und eines monoklonalen Antikörpers (Abcam, Cambridge, UK) nachgewiesen.

### 3. Kultivierung von Zellen

### a) Zelllinien

Die Zellkulturreagenzien wurden, wenn nicht anders angegeben, von der Firma Invitrogen GmbH bezogen. HEK293- und HeLa-Zellen wurden in Modifiziertem Eagles Medium (MEM) mit 10% fötalem Kälberserum (FCS), 1x Penicillin/Strepromycin bei 37°C, 95% Luftfuchtigkeit und 5% CO₂ kultiviert.

### b) Primäre Amniozyten

Primäre Amniozyten wurden, entsprechenden Routinemethoden folgend, im Rahmen einer Fruchtwasserpunktion gewonnen. Von dieser Punktion wurden 1-2 ml mit 5 ml Ham's F10 Medium, 10% FCS, 2% Ultroser G (CytoGen GmbH), 1x Antibiotikum/Antimycotikum, bei 37°C, 95% Luftfuchtigkeit und 5% CO₂ in 6-cm-Primaria Zellkulturschalen (Falcon) kultiviert. Nach 4-6 Tagen begannen die Amniozyten, adhärent zu werden, und es wurden 3 ml frisches Medium plus Zusätze (siehe oben) zugegeben. Waren die Zellen vollständig adhärent, wurde das Medium abgenommen und durch 5 ml frisches Medium plus Zusätze ersetzt. Für die weiteren Passagen wurden die konfluenten Zellen mit PBS gewaschen, mit Trypsin (TrypleSelect, Invitrogen) abgelöst und in 10 bzw. 25 ml frischem Medium plus Zusätze auf 10-cm- bzw.15-cm-Schalen übertragen.

### 4. Transfektion und Transformation primärer Amniozyten

Durch Transfektion verschiedener Kombinationen der oben beschriebenen Plasmide wurden primäre Amniozyten transfiziert, die transformierten Zelllinien isoliert und auf Expression des Transgens untersucht. Nachfolgend ist die Herstellung von hAAT-exprimierenden Zelllinien mit Hilfe der Plasmide pGS116 und pGS119 im Detail beschrieben. Weitere Zelllinien wurden auf dieselbe Weise mit folgenden Nukleinsäurekombinationen hergestellt:
- pSTK146 (enthält Ad5-Sequenzen nt. 505 bis 3522) und pGS116 (enthält die CMV-hAAT-Expressionskassette)
- pGS119 (enthält Ad5-Sequenzen nt. 505 bis 4079) und pGS129 (enthält die EF-1α-hAAT-Expressionskassette)
- pGS 119 und pGS131 (enthält die CAG-hAAT-Expressionskassette)
- pGS119 und pGS32 (enthält die RSV-hAAT-Expressionskassette)
- pGS 119 und pGS 133 (enthält die SV40-hAAT-Expressionskassette)
- pGS119 und pGS123 (enthält die CMV-hAAT-Expressionskassette und MAR-Sequenzen)
- pGS122 (enthält Ad5-Sequenzen nt. 1 bis 4344) und pGS116 (CMV-hAAT-Expressionskassette)
- pGS126 (enthält Ad5-Sequenzen nt. 505 bis 4079 und die CMV-hAAT-Expressionskassette)
- pGS119 und pGS127 (enthält die CMV-Epo-Expressionskassette)
- pGS124 (exprimiert T-Ag) und pGS116

Für die Transfektion wurden alle Plasmide außer pGS122 durch Verdau mit geeigneten Restriktionsnukleasen vorher linearisiert. Plasmid pGS122 wurde vor der Transfektion mit PmeI verdaut, da je eine PmeI-Schnittstelle die adenoviralen Sequenzen in pGS122 flankiert. Wurden zwei Plasmide transfiziert, so wurde von jedem Plasmid je 1 µg verwendet; bei Transfektion von nur einem Plasmid (pGS126) wurden 2 µg benutzt. Mit allen Nukleinsäurekombinationen konnten transformierte Zellklone erhalten und einzelne Klone isoliert und getestet werden. Die Anzahl der transformierten Zellklone war abhängig vom verwendeten Nukleinsäuremolekül, das den zell-transformierenden Faktor codierte. So wurden bei Verwendung der Plasmide pGS119 überraschender- und unerwarteterweise deutlich mehr transformierte Zellklone erhalten als mit pSTK146. Ferner führte die Transfektion mit Nukleinsäuremolekülen mit E1 als zelltransformierendem Faktor zu deutlich mehr transformierten Zellklonen als die Transfektion mit Nukleinsäuremolekülen, die das SV40 T-Antigen exprimierten (pGS124). Die Expression des rekombinanten Polypeptids war wiederum abhängig vom verwendeten Promotor, wobei die Verwendung des SV40-Promotors in pGS 133 die geringsten Expressionsraten ergab.

Nachfolgend ist die Herstellung von hAAT-exprimierenden Zelllinien mit Hilfe der Plasmide pGS 116 und pGS 119 im Detail beschrieben.

Vor der Transfektion wurden die Amniozyten schrittweise an Opti-Pro Medium mit 2% Ultroser adaptiert. Dazu wurden die Zellen immer nach jeweils 2-3 Tagen mit frischem Ham's F10 Medium (mit Zusätzen) plus Opti-Pro Medium (mit 2 % Ultroser) im Verhältnis 75:25%, 50:50%, 25:75% und 0:100% versetzt.
Für die Transfektion wurden die Zellen einer ca. 80% konfluenten 15-cm-Schale auf sechs 6-cm-Schalen verteilt, was einer Zellzahl von 5-7x10⁵ Zellen pro Schale entsprach. Am nächsten Tag wurden die Zellen auf 5 Schalen mit je 1 µg pGS116 und pGS119, beide linearisiert mit ScaI, unter Verwendung des Transfektionsreagenz Effectene (Qiagen) nach Angaben des Herstellers transfiziert. Eine Schale wurde untransfiziert als Kontrolle weiter kultivert. Am nächsten Tag wurden die Zellen mit PBS gewaschen, mit TrypleSelect abgelöst und auf je eine 15-cm-Schale übertragen. Die Zellen wurden für weitere 10-15 Tage kultiviert, wobei alle 3-4 Tage das Medium durch frisches Medium ersetzt wurde. Während dieser Zeit wurde der Zusatz von Ultroser auf 1% gesenkt. Nach ca. 10-15 Tagen waren die Zellen konfluent und wurden auf je zwei 15-cm-Schalen, wie oben beschrieben, umgesetzt.

### 5. Isolierung der transformierten Zellklone

Wenige Wochen nach der Transfektion waren klonale Zellinseln zu beobachten, die sich morphologisch deutlich von den nicht-transformierten Amniozyten unterschieden. Diese Zellinseln wurden gepickt und auf 24-well-Schalen übertragen (entspricht Passage 1). Die Zellen wurden weiter vermehrt und erst auf 6-cm-, danach auf 15-cm-Schalen übertragen.

Anfänglich wurden ca. 40 Klone isoliert, die sich bei weiterer Kultivierung zum Teil deutlich morphologisch unterschieden. Einige dieser Klone zeigten bei längerer Kultivierung eine deutliche "Crisis", d.h. sie verhielten sich in ihrem Wachstum sehr instabil. Die weiteren Experimente wurden auf die Weiterkultivierung und Analyse von sieben morphologisch stabilen Zellklonen beschränkt. Diese wurden wie folgt bezeichnet: 2AI.2, 2AI.3, 2AI.6. 2AI.12, 2AI.15, 2AI.17, 2AI.18.

### 6. Charakterisierung der Zelllinien

### a) Expression der E1-Gene (Western Blot)

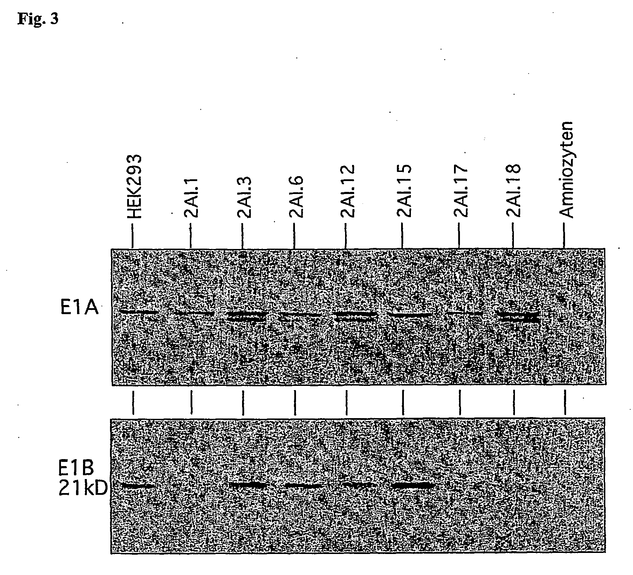

Die Expression der E1A- und EIB-21kD-Proteine in sieben klonalen Zelllinien wurden in Western Blot Analysen unter Verwendung monoklonaler Antikörper nachgewiesen.
Dazu wurden von den einzelnen Zellklonen 7x10⁵ Zellen in je einer 6-well-Schale ausplattiert. Zweiundsiebzig Stunden später wurden die Zellen mit Tris-Saline/4 mM EDTA abgelöst, pelletiert, in 100µl Tris-Saline aufgenommen und durch Zugabe von 30µl 4x SDS-Ladepuffer (40% Gylcerin, 1,4 M Mercaptoethanol, 8% SDS, 250 mM Tris/HCl pH 7) lysiert. Als Negativ-Kontrolle wurde Lysat verwendet, welches aus derselben Zahl primärer Amniozyten hergestellt worden war. Als Positiv-Kontrolle diente Lysat aus HEK293-Zellen. Von diesen Proteingemischen wurden je 10µl auf einem 10%-igen SDS-Polyacrylamidgel elektrophoretisch aufgetrennt und auf eine Nitrozellulosemembran transferiert. Die meinbrangebundenen Proteine wurden durch Inkubation mit einem Anti-EIA bzw. Anti-E1B-21kD-Antikörper (Oncogene Research) und einem HRP-gekoppelten Anti-Maus- (E1A, Jackson ImmunoResearch Laboratories) bzw. Anti-Ratte- (E1B-21kD, Oncogene Research) Antikörper und anschließender Inkubation mit Chemolumineszenz (ECL, Amersham) sichtbar gemacht. Die Ergebnisse der Western Blots mit E1A und E1B sind in Fig. 3 dargestellt und zeigen, dass alle untersuchten Zelllinien die E1A-Proteine (3 Banden bei 30 - 50 kD) und das E1B 21-kD-Protein exprimieren.

### b) hAAT-Expression (Western Blot)

Die Expression von hAAT wurde intrazellulär (in den klonalen Zelllinien) bzw. nach Sekretion (im Medium) ebenfalls mit Hilfe von Western Blot Analysen unter Verwendung eines monoklonalen hAAT-spezifischen Antikörpers nachgewiesen. Dazu wurden von den einzelnen Zellklonen 7x10⁵ Zellen in je eine 6-well-Schale plattiert. Als negative Kontrolle wurde dieselbe Zellzahl eines Zellklons verwendet, der durch Transfektion primärer Amniozyten nur mit pGS119 hergestellt worden war. Als positive Kontrolle dienten im anschließenden Assay 50 ng gereinigtes hAAT aus humanem Plasma.
Zweiundsiebzig Stunden später wurden die Zellkulturüberstande abgenommen, die Zellen mit Tris-Saline/4 mM EDTA abgelöst, pelletiert, in 100µl Tris-Saline aufgenommen und durch Zugabe von 30µl 4x SDS-Ladepuffer lysiert. Für den Nachweis des intrazellulären hAAT wurden 10µl des Proteingemisches der lysierten Zellen elektrophoretisch aufgetrennt und mit Hilfe eines monoklonalen hAAT-Antikörpers (ICN Biomedicals) und eines HRP-gekoppelten Anti-Ziege Antikörpers (Pierce) mit anschließender Chemolumineszenz-Färbung sichtbar gemacht. Zum Nachweis von sezerniertem hAAT wurden 10µl des Zellkulturmediums mit 2x SDS-Ladepuffer versetzt, elektrophoretisch aufgetrennt und wie oben beschrieben detektiert.

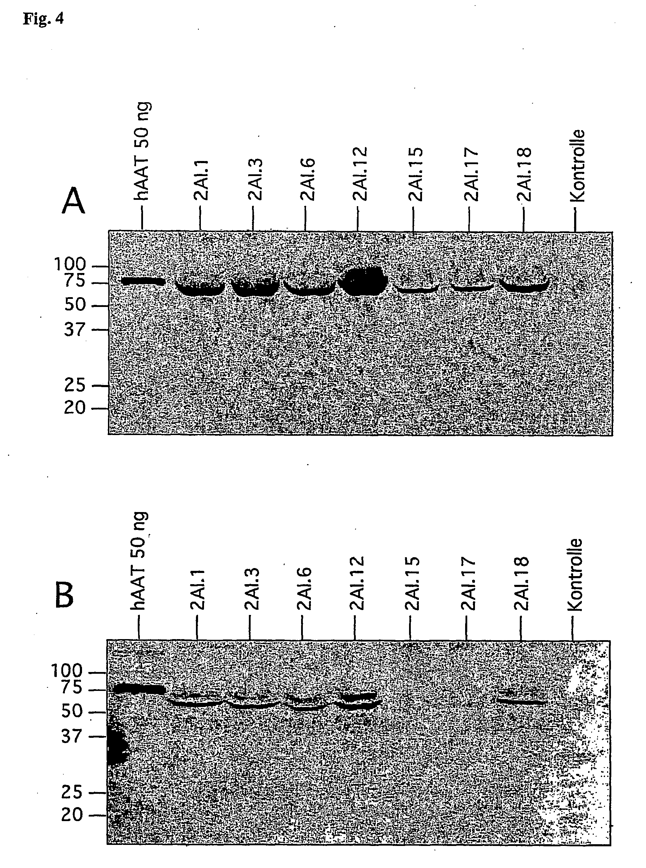

Fig. 4A zeigt das aus den Zellen in den Kulturüberstand sezernierte hAAT, die intrazelluläre Expression von hAAT ist in Fig. 4B dargestellt. Das Lysat für den intrazellulären Nachweis von hAATs ist im Gegensatz zum Zellkulturüberstand ca. 20-fach konzentriert. Dies bedeutet, dass hAAT sehr effizient von den Zellen sezerniert wird.

### c) Quantität und Stabilität der hAAT-Expression über mehrere Passagen (ELISA)

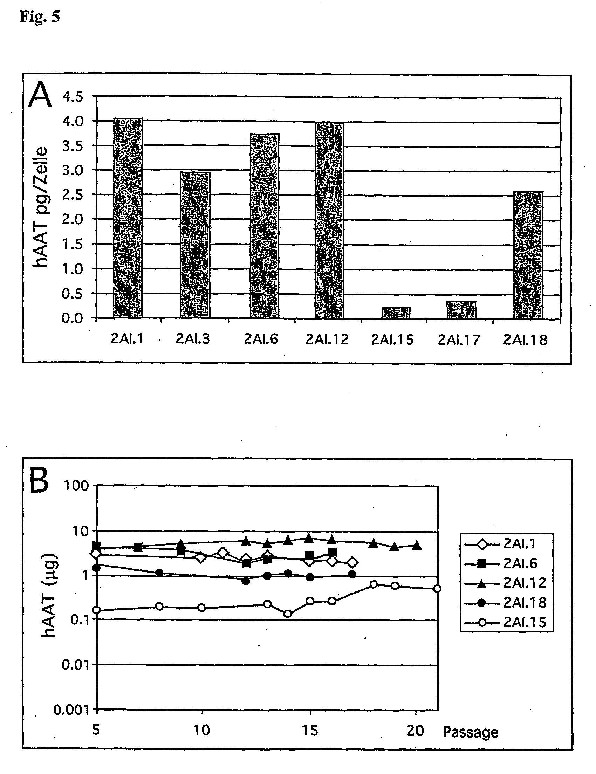

Die Menge des in das Medium sezernierten hAAT wurde quantitativ mit Hilfe der ELISA (Enzyme-linked Immunosorbent Assay)-Methode bestimmt. Dazu wurden 7x10⁵ Zellen der verschiedenen klonalen Linien in je eine 6-well-Schale plattiert und nach 72 Stunden wurden die Zellkulturüberstände abgenommen. Die Menge an hAAT wurde mittels ELISA unter Verwendung von polyklonalen Anti-hAAT-Antikörpern (ungekoppelt und HRP-gekoppelt; ICN Biomedicals) bestimmt. Als Kontrolle wurde aus humanem Plasma gereinigtes hAAT (ICN Biomedicals) verwendet. Fig. 5A zeigt die Menge an hAAT pro Zelle, exprimiert in Passage 5 der einzelnen Zellklone. Die Stabilität der Expression in ausgewählten Zellklonen über mehrere Passagen ist in Fig. 5B dargestellt. Die untersuchten Zelllinien exprimieren konstant über den erfaßten Zeitraum von ca. 20 Passagen zwischen 0.2 und 4 pg hAAT pro Zelle.

### d) Glykoanalyse von hAAT

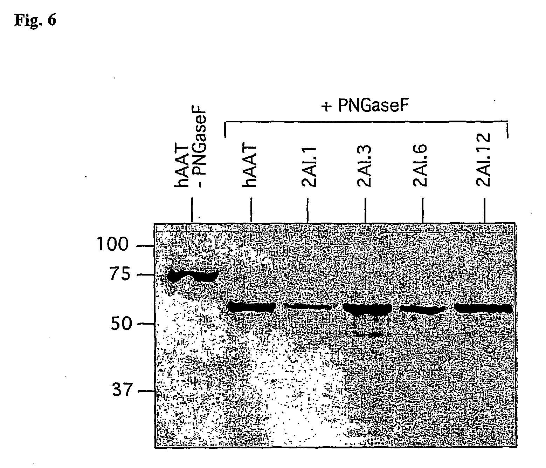

Da es sich bei hAAT um ein Glykoprotein handelt, wurde der Grad der Glykosylierung von hAAT in den einzelnen Zellklonen untersucht. Dazu wurden die Zellklone vorab schrittweise an Opti-Pro Medium ohne Ultroser adaptiert, was bewirkt, dass die Zellen nicht mehr adhärent sind, sondern in Suspension wachsen. Um zu testen, ob das in den Kulturüberstand sezernierte hAAT glykosyliert wird, wurde ein Verdau mit dem Enzym Peptid-N-Glykosidase F (PNGase F) durchgeführt. Dieses Enzym spaltet an Asparaginresten (N-linked) angehängte Zuckerreste ab, wodurch sich das Molekulargewicht des Proteins verringert. Von dem Zellkulturüberstand der einzelnen Zellklone wurden 22.5 µl mit 2.5 µl Deanturierungspuffer (10fach: 5% SDS, 10% β-Mercaptoethanol) versetzt und die Proteine für 10 Minuten bei 100°C denaturiert. Danach wurden den Proteinen je 3.5 µl G7-Reaktionspuffer (10fach: 500 mM Na-Phosphat pH 7.5), 3.5 µl 10% NP-40 und 3 µl PNGase F (New England Biolabs; 500 units/µl) zugegeben und für 60 Minuten bei 37°C inkubiert. Als Kontrollen wurden je 45 ng gereinigtes hAAT aus humanem Plasma in 25 µl Opti-Pro Medium wie oben beschrieben denaturiert und mit PNGase F bzw. H₂O versetzt. Die Veränderungen im Molekulargewicht von hAAT wurden abschließend wie unter 6b) beschrieben mittels Western Blot Analysen unter Verwendung eines monoklonalen hAAT-spezifischen Antikörpers nachgewiesen. Fig. 6 zeigt die Veränderung im Molkulargewicht nach Verdau mit PNGase F von hAAT, exprimiert in 4 verschiedenen Zellklonen, bzw. hAAT gereinigt aus humanem Plasma. Diese Experimente zeigen deutlich, dass das in den einzelnen Zellklonen produzierte hAAT glykosyliert ist.

## Patentansprüche

1. Verfahren zur Herstellung einer permanenten humanen Zelllinie, wobei isolierte Amniozyten mit
(i) einem Nukleinsäuremolekül oder
(ii) mindestens zwei verschiedenen Nukleinsäuremolekülen gleichzeitig transfiziert werden,
und falls ein Nulcleinsäuremolekül transfiziert wird, das Nukleinsäuremolekül eine Sequenz aufweist, die die Expression von E1A und E1B von humanen Adenoviren und die Expression eines rekombinanten Polypeptids erlaubt,
und falls mindestens zwei verschiedene Nukleinsäuremolelcüle transfiziert werden, ein erstes Nukleinsäuremolekül eine Sequenz aufweist, die die Expression von E1A und E1B von humanen Adenoviren erlaubt, und ein zweites Nukleinsäuremolekül eine Sequenz aufweist, die die Expression mindestens eines rekombinanten Polypeptids erlaubt.

2. Verfahren nach Anspruch 1, wobei die Sequenz für E1A und E1B von humanen Adenoviren die Nukleotide 1 bis 4344, 505 bis 3522 oder die Nukleotide 505 bis 4079 des humanen Adenovirus Serotyp 5 umfasst.

3. Verfahren nach einem der vorhergehenden Ansprüche, wobei das rekombinante Polypeptid ein Hormon, ein Blutgerinnungsfaktor, ein Wachstumsfaktor, der Coxsackie- und Adenovirus-Rezeptors (CAR), ein Antikörper, ein virales, bakterielles oder parasitäres Antigen, oder Komplementationsfaktoren zur Herstellung von rekombinanten Viren ist.

4. Verfahren nach einem der vorhergehenden Ansprüche, wobei die Sequenzen für E1A auf einem Nukleinsäuremolekül und die Sequenzen für E1B auf einem anderen verschiedenen Nukleinsäuremolekül liegen.

5. Verfahren nach Anspruch 4, wobei Sequenzen für das rekombinante Polypeptid auf einem der beiden Nukleinsäuremoleküle für E1A und E1B liegt.

6. Verfahren nach einem der vorhergehenden Ansprüche, wobei die Sequenz, die die Expression von E1A und E1B oder eines rekombinanten Polypeptids erlaubt, einen heterologen Promotor und/oder ein heterologes transkriptionelles Terminationselement aufweist.

7. Verfahren nach Anspruch 6, wobei der Promotor der CMV- (Cytomegalievirus-) Promotor, EF-1α-Promotor, CAG-Promotor, humaner oder muriner pgk-Promotor, RSV-Promotor oder SV40-Promotor ist, und das transkriptionelle Terminationselement die Polyadenylierungssequenzen des SV40 Large T-Antigens oder des humanen G-CSF-Gens ist.

8. Verfahren nach einem der vorhergehenden Ansprüche, wobei die Sequenz, die die Expression des rekombinanten Polypeptids erlaubt, Erkennungssequenzen für Rekombinasen enthält, die die codierende Sequenz des rekombinanten Polypeptids oder die vollständige Expressionskassette des rekombinanten Polypeptids flankieren.

9. Verfahren nach Anspruch 8, wobei die Erkennungssequenzen loxP-, attB/attP- oder Frt-Sequenzen sind.

10. Verfahren nach einem der vorhergehenden Ansprüche, wobei die transfizierten Nukleinsäuremoleküle keine Sequenzen aufweisen, die einen Selektionsmarker codieren.

11. Permanente humane Zelllinien, erhältlich nach einem Verfahren nach einem der Ansprüche 1 bis 10, wobei das Verfahren ohne Transfektion eines Selektionsmarkers durchgeführt wurde.

12. Verwendung der Zelllinien nach Anspruch 11 für die Herstellung eines Polypeptids.

13. Verwendung der Zelllinien nach Anspruch 11 für die Herstellung von viralen Gentransfer-Vektoren.

## Claims

1. Method for production of a permanent human cell line, wherein isolated amniocytes are transfected with
(i) a nucleic acid molecule or
(ii) at least two distinct nucleic acid molecules simultaneously,
and if a nucleic acid molecule is transfected, the nucleic acid molecule exhibits a sequence allowing the expression of E1A and E1B of human adenoviruses and the expression of a recombinant polypeptide,
and if at least two distinct nucleic acid molecules are transfected, a first nucleic acid molecule exhibits a sequence allowing the expression of E1A and E1B of human adenoviruses, and a second nucleic acid molecule exhibits a sequence allowing the expression of at least one recombinant polypeptide.

2. Method according to claim 1, wherein the sequence for E1A and E1B of human adenoviruses comprises the nucleotides 1 to 4344, 505 to 3522 or the nucleotides 505 to 4079 of the human adenovirus serotype 5.

3. Method according to any one of the preceding claims, wherein the recombinant polypeptide is a Hormone, a blood coagulation factor, a growth factor, the Coxsackie- and adenovirus receptor (CAR), an antibody, a viral, bacterial or parasitic antigen, or complementation factors for production of recombinant viruses.

4. Method according to any one of the preceding claims, wherein the sequences for E1A are present on one nucleic acid molecule and the sequences for E1B are present on another distinct nucleic acid molecule.

5. Method according to claim 4, wherein sequences of the recombinant polypeptide are present on one of the two nucleic acid molecules for E1A and E1B.

6. Method according to any one of the preceding claims, wherein the sequence allowing the expression of E1A and E1B or of a recombinant polypeptide, exhibits a heterologous promoter and/or a heterologous transcriptional termination element.

7. Method according to claim 6, wherein the promoter is the CMV (cytomegalovirus) promoter, EF-1α promoter, CAG promoter, human or murine pgk promoter, RSV promoter or SV40 promoter, and the transcriptional termination element is the polyadenylation sequences of the SV40 Large T-antigen or of the human G-CSF gene.

8. Method according to any one of the preceding claims, wherein the sequence allowing the expression of the recombinant polypeptide contains recognition sequences for recombinases flanking the coding sequence of the recombinant polypeptide or the complete expression cassette of the recombinant polypeptide.

9. Method according to claim 8, wherein the recognition sequences are loxP, attB/attP or Frt sequences.

10. Method according to any one of the preceding claims, wherein the transfected nucleic acid molecules exhibit no sequences encoding a selection marker.

11. Permanent human cell lines, obtainable according to a method according to any one of claims 1 to 10, wherein the method was performed without transfection of a selection marker.

12. Use of the cell lines according to claim 11 for the production of a polypeptide.

13. Use of the cell lines according to claim 11 for the production of viral gene transfer vectors.

## Revendications

1. Procédé de fabrication d'une lignée cellulaire humaine permanente, dans lequel des amniocytes isolés sont transfectés avec
(i) une molécule d'acide nucléique ou
(ii) au moins deux molécules d'acide nucléique différentes simultanément,
et, dans le cas où une molécule d'acide nucléique est transfectée, la molécule d'acide nucléique présente une séquence qui permet l'expression de E1A et E1B d'adénovirus humains et l'expression d'un polypeptide recombinant,
et, dans le cas où au moins deux molécules d'acide nucléique différentes sont transfectées, une première molécule d'acide nucléique présente une séquence qui permet l'expression de E1A et E1B d'adénovirus humains et une deuxième molécule d'acide nucléique présente une séquence qui permet l'expression d'au moins un polypeptide recombinant.

2. Procédé selon la revendication 1 dans lequel la séquence pour E1A et E1B d'adénovirus humains comprend les nucléotides 1 à 4 344, 505 à 3 522 ou les nucléotides 505 à 4 079 de l'adénovirus humain de sérotype 5.

3. Procédé selon l'une des revendications précédentes, dans lequel le polypeptide recombinant est une Hormone, un facteur de coagulation sanguine, un facteur de croissance, le récepteur au virus Coxsackie et à l'adénovirus (CAR), un anticorps, un antigène viral, bactérien ou parasitaire, ou des facteurs de complémentation pour la fabrication de virus recombinants.

4. Procédé selon l'une des revendications précédentes, dans lequel les séquences pour E1A se trouvent dans une molécule d'acide nucléique et les séquences pour E1B se trouvent dans une autre molécule d'acide nucléique différente.

5. Procédé selon la revendication 4, dans lequel les séquences pour le polypeptide recombinant se trouvent dans l'une des deux molécules d'acide nucléique pour E1A et E1B.

6. Procédé selon l'une des revendications précédentes dans lequel la séquence qui permet l'expression de E1A et E1B ou d'un polypeptide recombinant présente un promoteur hétérologue et/ou un élément d'arrêt de la transcription hétérologue.

7. Procédé selon la revendication 6, dans lequel le promoteur est le promoteur du CMV (cytomégalovirus), le promoteur d'EF-1α, le promoteur CAG, le promoteur pgk humain ou murin, le promoteur du RSV ou le promoteur du SV40 et l'élément d'arrêt de la transcription est la séquence de polyadénylation de l'antigène grand T du SV40 ou du gène du G-CSF humain.

8. Procédé selon l'une des revendications précédentes dans lequel la séquence qui permet l'expression du polypeptide recombinant contient des séquences de reconnaissance de recombinases qui encadrent la séquence codante du polypeptide recombinant ou la cassette d'expression complète du polypeptide recombinant.

9. Procédé selon la revendication 8, dans lequel les séquences de reconnaissance sont les séquences loxP, attB/attP ou Frt.

10. Procédé selon l'une des revendications précédentes, dans lequel les molécules d'acide nucléique transfectées ne présentent aucune séquence codant pour un marqueur de sélection.

11. Lignées cellulaires humaines permanentes obtenues selon un procédé selon l'une des revendications 1 à 10, le procédé ayant été réalisé sans transfection d'un marqueur de sélection.

12. Utilisation des lignées cellulaires selon la revendication 11 pour la fabrication d'un polypeptide.

13. Utilisation des lignées cellulaires selon la revendication 11 pour la fabrication de vecteurs de transfert de gènes viraux.
